# FASCICULE DE BREVET EUROPEEN

(11) **EP 0 855 897 B1**
(45) Date de publication et mention de la délivrance du brevet: **09.02.2005**
(21) Numéro de dépôt: 96933505.8
(22) Date de dépôt: 08.10.1996
(51) Int. Cl.: A61K 7/06

(54) **LAQUE A L'EAU POUR LE TRAITEMENT DES MATIERES KERATINIQUES, CONDITIONNEE DANS UN DISPOSITIF AEROSOL, COMPRENANT AU MOINS UN POLYMERE SILICONE GREFFE ET APPLICATIONS**
YACK AUF WASSERBASIS FÜR DIE BEHANDLUNG VON KERATINMATERIALIEN, DER IN EINEM AEROSOL-BEHÄLTER VERPACKT IST, DER MINDESTENS EIN GEPFROPFTES SILICONPOLYMER ENTHÄLT, UND DESSEN VERWENDUNGEN
AQUEOUS LACQUER PACKAGED IN AN AEROSOL DEVICE AND INCLUDING AT LEAST ONE SILICONE GRAFT POLYMER FOR TREATING KERATINOUS MATERIALS, AND USES THEREOF

(30) Priorité: 18.10.1995 FR 9512234
(43) Date de publication de la demande: 05.08.1998
(73) Titulaire: L'OREAL, 75008 Paris (FR)
(72) Inventeur: DUPUIS, Christine, F-75018 Paris (FR)
(74) Mandataire: Miszputen, Laurent
(86) Numéro de dépôt international: PCT/FR1996/001571
(87) Numéro de publication internationale: WO 1997/014395

(56) Documents cités:
- EP-A- 0 408 311
- EP-A- 0 524 612
- WO-A-93/03703
- WO-A-95/05800
- FR-A- 2 709 955

## Description

La présente invention a trait à une composition aqueuse pour le traitement des matières kératiniques, en particulier des cheveux humains, sous forme de laque, conditionnée dans un dispositif aérosol pour produire une laque, comprenant une faible quantité de solvant organique et au moins un polymère siliconé greffé comprenant une portion polysiloxane et une portion constituée d'une chaîne organique non-siliconée, l'une des deux portions constituant la chaîne principale du polymère, l'autre étant greffée sur ladite chaîne principale.

Dans ce qui suit, on entend par «laque» toute composition sortant à la sortie d'un dispositif de pulvérisation sous forme de gouttelettes liquides directement projetées sur le support kératinique.

Les polymères du type polymère siliconé greffé comprenant une portion polysiloxane et une portion constituée d'une chaîne organique non-siliconée, l'une des deux portions constituant la chaîne principale du polymère l'autre étant greffée sur ladite chaîne principale sont connus pour leurs propriétés coiffantes. Ils sont particulièrement intéressants en cosmétique capillaire du fait qu'ils apportent de la tenue aux cheveux.

Depuis un certain nombre d'années, un intérêt tout particulier s'est manifesté pour les laques aérosols comme mode de conditionnement de formulations pour le maintien de la coiffure et/ou la mise en forme de la coiffure. Les laques aérosols contenant les polymères siliconés greffés tels que décrits ci-dessus, sont généralement alcooliques et plus préférentiellement hydroalcooliques et contiennent généralement en plus un tensio-actif. Ces compositions, contenant plus de 25% en poids de solvant organique, ont tendance, après dépôt desdits polymères de conduire à des phénomènes de mousse, de conférer des propriétés de brillance insuffisantes de donner des pouvoirs fixants non totalement suffisants et d'alourdir la chevelure (effets de charge).

La demanderesse a trouvé de façon surprenante que l'utilisation d'un polymère tel que défini dans la formule (IV), pressurisée dans un dispositif aérosol, comprenant au moins 30% en poids d'eau et au plus 25% en poids de solvant organique, après application et séchage, permettait d'améliorer la brillance des cheveux et de présenter un meilleur pouvoir laquant. En outre, cette utilisation permettait de réduire sensiblement voire de supprimer totalement les effets de charge sur les cheveux.

La demanderesse a constaté également que les laques aqueuses pressurisées conformes à l'invention ne conduisaient pas à des phénomènes de mousse sur les cheveux entraînant un effet blanchissant ni à des phénomènes de poudrage ou de poissage. Elles apportent en outre de bonnes propriétés de coiffage et de recoiffage, une bonne tenue à la chevelure ainsi qu'un démêlage facile.

La composition aqueuse, selon la présente invention, conditionnée dans un dispositif aérosol et produisant à la sortie du dispositif une laque est caractérisée par le fait qu'elle comprend :
(a) au moins un polymère siliconé tel que défini dans la formule IV
(b) au moins 30% en poids d'eau ;
(c) au moins un agent propulseur ;
(d) une quantité de solvant organique allant de 0 à 25% en poids ; les pourcentages en poids étant définis par rapport au poids total de la composition dans l'aérosol.

Les polymères siliconés comportent dans leur structure le motif de formule (IV) suivant: 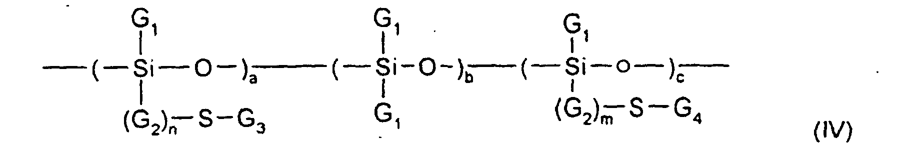 dans lequel les radicaux G₁, identiques ou différents, représentent l'hydrogène ou un radical alkyle en C₁-C₁₀ ou encore un radical phényle ; les radicaux G₂, identiques ou différents, représentent un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère hydrophobe à insaturation éthylénique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

De préférence, le motif de formule (IV) ci-dessus présente au moins l'une, et encore plus préférentiellement l'ensemble, des caractéristiques suivantes :
- les radicaux G₁ désignent un radical alkyle, de préférence le radical méthyle ;
- n est non nul, et les radicaux G₂ représentent un radical divalent en C₁-C₃, de préférence un radical propylène ;
- G₃ représente un radical polymérique résultant de l'(homo)polymérisation d'au moins un monomère du type acide carboxylique à insaturation éthylénique, de préférence l'acide acrylique et/ou l'acide méthacrylique ;
- G₄ représente un radical polymérique résultant de l'(homo)polymérisation d'au moins un monomère du type (méth)acrylate d'alkyle(C₁-C₁₀), de préférence le (méth)acrylate d'isobutyle ou de méthyle.

Des exemples de polymères siliconés greffés répondant à la formule (IV) sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth) acrylate de méthyle.

D'autres exemples de polymères siliconés greffés répondant à la formule (IV) sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

De préférence, la masse moléculaire en nombre des polymères siliconés à squelette polysiloxanique greffé par des monomères organiques non-siliconés de l'invention varie de 10 000 à 1 000 000 environ, et encore plus préférentiellement de 10 000 à 100 000 environ.

Les polymères greffés siliconés de l'invention sont utilisés de préférence en une quantité allant de 0,01 à 20% en poids du poids total de la composition. Plus préférentiellement, cette quantité varie de 0,1 à 15% en poids et encore plus préférentiellement de 0,5 à 10% en poids.

Les polymères siliconés greffés de l'invention peuvent être dissous dans le milieu aqueux de la laque aérosol ou utilisés sous forme de dispersion aqueuse de particules .

Les compositions selon l'invention contiennent de préférence de 45 à 100% d'eau par rapport au poids total de la composition dans l'aérosol et plus particulièrement de 60 à 99,5% d'eau.

Les solvants organiques utilisables dans les compositions aérosols de l'invention, sont choisis de préférence parmi les alcools en C₁-C₄ tels que par exemple l'éthanol, l'isopropanol.

Les solvants organiques sont présents dans les compositions aérosols dans des concentrations allant préférentiellement jusqu'à 10% en poids par rapport au poids total de la composition dans l'aérosol et plus particulièrement jusqu'à 5% en poids. Le cas corresoôndant à l'absence totale de solvant organique est tout particulièrement avantageux.

Les compositions selon l'invention, conditionnées sous forme d'aérosol en vue d'obtenir une laque, comprennent un agent propulseur qui peut être choisi dans le groupe constitué par les hydrocarbures volatils tels que le n-butane, le propane, l'isobutane, le pentane ; les hydrocarbures chlorés et/ou fluorés et leurs mélanges. On peut également utiliser en tant qu'agent propulseur le gaz carbonique, le protoxyde d'azote, le diméthyléther, l'azote, l'air comprimé et leurs mélanges. Le diméthyléther est particulièrement préféré.

La concentration du gaz propulseur dans le dispositif aérosol dépend de la nature du propulseur choisi.Il est utilisé dans les compositions aérosols de l'invention dans des concentrations allant de préférence de 10 à 68% en poids par rapport au poids total de la composition dans le dispositif aérosol et plus particulièrement de 25 à 50% en poids.

La concentration en composé organique volatil (COV) dans une composition selon l'invention conditionnée sous forme d'aérosol est de préférence inférieure ou égale à 55% en poids par rapport au poids total de la formulation conditionée en aérosol.

Le pH des compositions selon l'invention est généralement compris entre 2 et 9 et en particulier entre 3 et 8. Il peut être ajusté à la valeur choisie au moyen d'agents alcanisants ou acidifiants habituellement utilisés en cosmétique.

Les compositions capillaires conformes à l'invention peuvent contenir en plus des additifs cosmétiques conventionnels tels que des conservateurs, des adoucissants, des séquestrants, des parfums, des modificateurs de viscosité, des agents nacrants, des agents hydratants, des agents antipelliculaires, des agents anti-séborrhéiques, des filtres solaires, des agents de conditionnement pour les cheveux, des anti-oxydants, des protéines, des vitamines, des silicones, des polymères. En particulier, les compositions de l'invention peuvent contenir en plus des polymères coiffants à propriétés de fixation de la chevelure, anioniques, cationiques, amphotères ou non-ioniques.

Un des avantages des compositions selon l'invention par rapport aux laques aérosols aqueuses de l'art antérieur contenant des polymères greffés siliconés est qu'elles peuvent être exemptes de tout tensioactif.

Les compositions selon l'invention telles que définies ci-dessus peuvent être utilisées comme produit coiffant pour la mise en forme et/ou le maintien de la coiffure et/ou pour le conditionnement des cheveux.

Un autre objet de l'invention consiste en un procédé non-thérapeutique pour mettre en forme et/ou maintenir les cheveux et/ou pour le conditionnement des cheveux, caractérisé en ce qu'il consiste à appliquer directement sur les cheveux une composition telle que définie précédemment.

Les exemples qui suivent servent à illustrer l'invention sans toutefois présenter un caractère limitatif.

### EXEMPLE 1 Laque aerosol à 55% de COV (Composé Organique Volatile)

- Polymère siliconé greffé de formule (IV) de structure polyméthyl/méthylsiloxane à groupements propyl thio-3 acide polyméthacrylique et groupements propyl thio-3 polyméthacrylate de méthyle 5 g en MA
- Aminométhylpropanol neutralisation à 100% qsp
- Ethanol 20 g
- Eau déminéralisée 40 g
- Diméthyléther 35 g

### EXEMPLE 2 Laque aérosol à 30% de COV

- Polymère siliconé greffé de formule (IV) de structure polyméthyl/méthylsiloxane à groupements propyl thio-3 acide polyméthacrylique et groupements propyl thio-3 polyméthacrylate de méthyle 7 g en MA
- Aminométhylpropanol neutralisation à 100% qsp
- Eau déminéralisée 63 g
- Diméthyléther 30 g

### EXEMPLE 3 Laque aérosol à 55% de COV

- Polymère siliconé greffé de structure (1) telle que définie ci-dessous 3 g en MA
- Aminométhylpropanol qsp
- Ethanol 17 g
- Eau déminéralisée 42 g
- Diméthyléther 38 g

### Structure (1) :

Copolymère obtenu par polymérisation radicalaire à partir du mélange de monomères constitué par :
a) 60% en poids d'acrylate de tertiobutyle ;
b) 20% en poids d'acide acrylique ;
c) 20% en poids de macromère siliconé de formule : 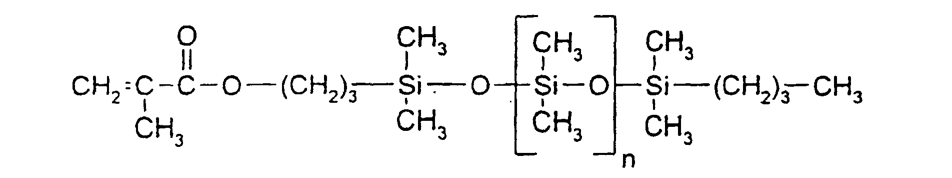
avec n étant un nombre choisi de telle sorte que le poids moléculaire moyen en nombre du macromère soit compris entre environ 9.000 et 12.000 ; les pourcentages en poids étant calculés par rapport au poids total des monomères.

### EXEMPLES COMPARATIES

On compare les 4 formulations de laques à l'eau aérosol A, B, C, et D défini dans le tableau suivant:

| **Formulation** | **A (art antérieur)** | **B (invention)** | **C (invention)** | **D (invention)** |
|---|---|---|---|---|
| Polymère greffé siliconé des exemples 1 et 2 | 3 g MA | 3 g MA | 3 g MA | 3 g MA |
| Aminométhyl propanol | qs neutralisation 100% | qs neutralisation 100% | qs neutralisation 100% | qs neutralisation 100% |
| Eau | 20 g | 42 g | 42 g | 67 g |
| Ethanol | 47 g | 25 g | 25 g | - |
| Diméthyléther | 15 g | 15 g | 30 | 30 g |
| Mélange isobutane/ propane/butane (23/55/22) | 15 g | 15 g | - | - |

On a effectué un test d'évaluation sensorielle sur un panel de 5 personnes. On étudie pour chaque laque aérosol A, B, C et D, comme critère cosmétique le pouvoir laquant sur les cheveux après application et séchage , l'effet de poudrage après démêlage des cheveux fixés, la brillance après démêlage.

On effectue, avec chaque laque testée, une vaporisation pendant 10 s sur un échantillon de mèches de cheveux de 5 g posées en éventail sur un support.

Les 5 personnes interrogées ont estimé que les compositions B, C et D selon l'invention contenant plus de 40% en poids d'eau et au plus 25% en poids de solvant organique présentaient un meilleur pouvoir laquant, une meilleure brillance et un effet de poudrage moindre par rapport à la composition A selon l'art antérieur contenant plus de 25% en poids de solvant organique (47%). De plus, la laque A conduit après pulvérisation à des traces blanches sur les cheveux au séchage (effet de mousse) contrairement aux compositions B, C et D de l'invention.

## Revendications

1. Composition aqueuse pour le traitement des matières kératiniques, conditionnée dans un dispositif aérosol, produisant une laque à la sortie dudit dispositif, **caractérisée par le fait qu'**elle comprend :
(a) au moins un polymère siliconé greffé à squelette polysiloxanique greffé par des monomères organiques non-siliconés;
(b) au moins 30% en poids d'eau ;
(c) au moins un agent propulseur ;
(d) une quantité de solvant organique allant de 0 à 25% en poids ; les pourcentages en poids étant définis par rapport au poids total de la composition dans l'aérosol, et
le polymère siliconé greffé (a) est choisi parmi les polymères siliconés comportant dans leur structure le motif de formule (IV) suivant : 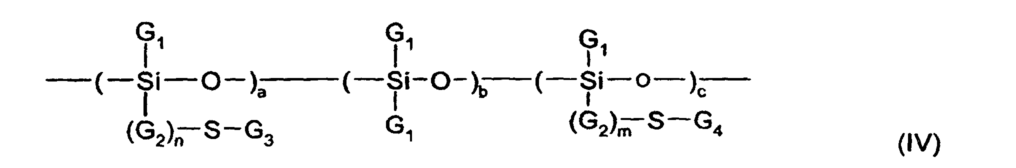 dans lequel les radicaux G₁, identiques ou différents, représentent l'hydrogène ou un radical alkyle en C₁-C₁₀ ou encore un radical phényle ; les radicaux G₂, identiques ou différents, représentent représente un groupe alkylène en C₁-C₁₀ ; G₃ représente un reste polymérique résultant de l'(homo)polymérisation d'au moins un monomère anionique à insaturation éthylénique ; G₄ représente un reste polymérique résultant de l'(homo)polymérisation d'au moins un monomère hydrophobe à insaturation éthylènique ; m et n sont égaux à 0 ou 1 ; a est un nombre entier allant de 0 et 50 ; b est un nombre entier pouvant être compris entre 10 et 350, c est un nombre entier allant de 0 et 50 ; sous réserve que l'un des paramètres a et c soit différent de 0.

2. Composition selon la revendication 1, **caractérisée par le fait que** le motif de formule (IV) présente au moins l'une des caractéristiques suivantes :
- les radicaux G₁ désignent un radical alkyle en C₁-C₁₀ ;
- n est non nul, et les radicaux G₂ représentent un radical divalent en C₁-C₃ ;
- G₃ représente un radical polymérique résultant de l'(homo)polymérisation d'au moins un monomère du type acide carboxylique à insaturation éthylénique ;
- G₄ représente un radical polymérique résultant de l'(homo)polymérisation d'au moins un monomère du type (méth)acrylate d'alkyle (C₁-C₁₀).

3. Composition selon la revendication 1 ou 2, **caractérisée par le fait que** le motif de formule (IV) présente simultanément les caractéristiques suivantes :
- les radicaux G₁ désignent un radical méthyle ;
- n est non nul, et les radicaux G₂ représentent un radical propylène ;
- G₃ représente un radical polymérique résultant de l'(homo)polymérisation d'au moins l'acide acrylique et/ou l'acide méthacrylique ;
- G₄ représente un radical polymérique résultant de l'(homo)polymérisation d'au moins un monomère du type (méth)acrylate d'isobuyle ou de méthyle.

4. Composition selon l'une quelconque des revendications 1 à 3, **caractérisée par le fait que** la masse moléculaire en nombre du polymère à squelette polysiloxanique greffé par des monomères organiques non-siliconés varie de 10 000 à 1 000 000 environ, et encore plus préférentiellement de 10 000 à 100 000 environ.

5. Composition selon l'une quelconque des revendications 1 à 4, **caractérisée par le fait que** le ou les polymères siliconés greffés sont utilisés en une quantité allant de 0,01 à 20% en poids par rapport au poids total de la composition et de préférence de 0,1 à 15% en poids et encore plus préférentiellement de 0,5 à 10% en poids.

6. Composition selon l'une quelconque des revendications 1 à 5, **caractérisée par le fait qu'**elle contient de 45 à 100% d'eau par rapport au poids total de la composition dans l'aérosol et plus préférentiellement de 60 à 99,5% d'eau.

7. Composition selon l'une quelconque des revendications 1 à 6, **caractérisée par le fait que** le solvant organique est choisi parmi les alcools en C₁-C₄.

8. Composition selon l'une quelconque des revendications 1 à 7, **caractérisée par le fait qu'**elle contient de 0 à 10% de solvant organique par rapport au poids total de la composition dans l'aérosol et plus préférentiellement de 0 à 5% de solvant organique.

9. Composition selon l'une quelconque des revendications 1 à 8, **caractérisée par le fait qu'**elle ne contient pas de solvant organique.

10. Composition selon l'une quelconque des revendications 1 à 9, **caractérisée par le fait que** l'agent propulseur est choisi dans le groupe constitué par les hydrocarbures volatils ; les hydrocarbures chlorés et/ou fluorés et leurs mélanges ; le gaz carbonique, le protoxyde d'azote, le diméthyléther, l'azote, l'air comprimé et leurs mélanges.

11. Composition selon l'une quelconque des revendications 1 à 10, **caractérisée par le fait que** l'agent propulseur est le diméthyléther.

12. Composition selon l'une quelconque des revendications 1 à 11, **caractérisée par le fait que** l'agent propulseur est présent dans des concentrations allant de préférence de 10 à 68% en poids par rapport au poids total de la composition dans le dispositif aérosol et plus particulièrement allant de 25 à 50% en poids.

13. Composition selon l'une quelconque des revendications 1 à 12, **caractérisée par le fait que** la concentration en composé organique volatil (COV) est inférieure ou égale à 55% en poids par rapport au poids total de la composition conditionnée en aérosol.

14. Composition selon l'une quelconque des revendications 1 à 13, **caractérisée par le fait que** le pH varie de 2 à 9 et en particulier de 3 à 8.

15. Composition selon l'une quelconque des revendications 1 à 14, **caractérisée par le fait qu'**elle contient en plus un additif cosmétique conventionnel choisi dans le groupe constitué par des conservateurs, des adoucissants, des séquestrants, des parfum, des modificateurs de viscosité, des agents nacrants, des agents hydratants, des agents antipelliculaires, des agents anti-séborrhéiques, des filtres solaires, des agents de conditionnement pour les cheveux, des anti-oxydants, des protéines, des vitamines, des silicones, des agents alcanisants ou acidifiants des polymères anioniques, cationiques, amphotères ou non-ioniques coiffants à propriétés de fixation de la chevelure, leurs mélanges.

16. Composition selon l'une quelconque des revendications 1 à 15, **caractérisée par le fait que** le polymère siliconé greffé est dissous dans le milieu aqueux de la composition ou utilisé sous forme de dispersion aqueuse de particules.

17. Composition selon l'une quelconque des revendications 1 à 16, **caractérisée par le fait que** les matières kératiniques sont des cheveux humains.

18. Composition selon l'une quelconque des revendications 1 à 17, **caractérisée par le fait qu'**elle est un produit capillaire pour le maintien et/ou la mise en forme de la chevelure et/ou pour le conditionnement des cheveux.

19. Procédé non-thérapeutique de maintien et/ou de mise en forme de la chevelure et/ou de conditionnement des cheveux, **caractérisé par le fait qu'**il consiste à appliquer sur les cheveux une composition telle que définie selon l'une quelconque des revendications 1 à 18.

## Claims

1. Aqueous composition for treating keratin substances, packaged in an aerosol device and producing a lacquer at the outlet of the said device, **characterized in that** it comprises:
(a) at least one grafted silicone polymer containing a polysiloxane skeleton grafted with non-silicone organic monomers;
(b) at least 30% by weight of water;
(c) at least one propellant;
(d) an amount of organic solvent ranging from 0 to 25%
by weight; the weight percentages being defined relative to the total weight of the composition in the aerosol, and
the grafted silicone polymer (a) is chosen from silicone polymers containing in their structure the unit of formula (IV) below: 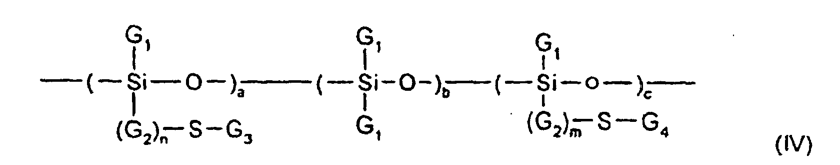 in which the radicals G₁, which may be identical or different, represent hydrogen or a C₁-C₁₀ alkyl radical or alternatively a phenyl radical; the radicals G₂, which may be identical or different, represent a C₁-C₁₀ alkylene group; G₃ represents a polymer residue resulting from the (homo)polymerization of at least one anionic monomer containing ethylenic unsaturation; G₄ represents a polymer residue resulting from the (homo)polymerization of at least one hydrophobic monomer containing ethylenic unsaturation; m and n are equal to 0 or 1; a is an integer ranging from 0 to 50; b is an integer which may be between 10 and 350, c is an integer ranging from 0 to 50; with the proviso that one of the parameters a and c is other than 0.

2. Composition according to Claim 1, **characterized in that** the unit of formula (IV) has at least one of the following characteristics:
- the radicals G₁ denote a C₁-C₁₀ alkyl radical;
- n is non-zero and the radicals G₂ represent a divalent C₁-C₃ radical;
- G₃ represents a polymeric radical resulting from the (homo)polymerization of at least one monomer of the carboxylic acid type containing ethylenic unsaturation;
- G₄ represents a polymeric radical resulting from the (homo)polymerization of at least one monomer of the C₁-C₁₀ alkyl (meth)acrylate type.

3. Composition according to Claim 1 or 2, **characterized in that** the unit of formula (IV) simultaneously has the following characteristics:
- the radicals G₁ denote a methyl radical;
- n is non-zero and the radicals G₂ represent a propylene radical;
- G₃ represents a polymeric radical resulting from the (homo)polymerization of at least acrylic acid and/or methacrylic acid;
- G₄ represents a polymeric radical resulting from the (homo)polymerization of at least one monomer of the isobutyl or methyl (meth)acrylate type.

4. Composition according to any one of Claims 1 to 3, **characterized in that** the number-average molecular mass of the polymer containing a polysiloxane skeleton grafted with non-silicone organic monomers ranges approximately from 10,000 to 1,000,000 and even more preferably approximately from 10,000 to 100,000.

5. Composition according to any one of Claims 1 to 4, **characterized in that** the grafted silicone polymer(s) is (are) used in an amount ranging from 0.01 to 20% by weight relative to the total weight of the composition and preferably from 0.1 to 15% by weight and even more preferably from 0.5 to 10% by weight.

6. Composition according to any one of Claims 1 to 5, **characterized in that** it contains from 45 to 100% of water relative to the total weight of the composition in the aerosol, and more preferably from 60 to 99.5% of water.

7. Composition according to any one of Claims 1 to 6, **characterized in that** the organic solvent is chosen from C₁-C₄ alcohols.

8. Composition according to any one of Claims 1 to 7, **characterized in that** it contains from 0 to 10% of organic solvent relative to the total weight of the composition in the aerosol, and more preferably from 0 to 5% of organic solvent.

9. Composition according to any one of Claims 1 to 8, **characterized in that** it contains no organic solvent.

10. Composition according to any one of Claims 1 to 9, **characterized in that** the propellant is chosen from the group consisting of volatile hydrocarbons; chlorinated and/or fluorinated hydrocarbons and mixtures thereof; carbon dioxide, nitrous oxide, dimethyl ether, nitrogen, compressed air and mixtures thereof.

11. Composition according to any one of Claims 1 to 10, **characterized in that** the propellant is dimethyl ether.

12. Composition according to any one of Claims 1 to 11, **characterized in that** the propellant is present in concentrations preferably ranging from 10 to 68% by weight relative to the total weight of the composition in the aerosol device, and more particularly ranging from 25 to 50% by weight.

13. Composition according to any one of Claims 1 to 12, **characterized in that** the concentration of volatile organic compound (VOC) is less than or equal to 55% by weight relative to the total weight of the composition packaged as an aerosol.

14. Composition according to any one of Claims 1 to 13, **characterized in that** the pH ranges from 2 to 9 and in particular from 3 to 8.

15. Composition according to any one of Claims 1 to 14, **characterized in that** it also contains a conventional cosmetic additive chosen from the group consisting of preserving agents, softeners, sequestering agents, fragrances, viscosity modifiers, pearlescent agents, moisturizers, antidandruff agents, antiseborrhoeic agents, sunscreens, hair conditioners, antioxidants, proteins, vitamins, silicones, basifying or acidifying agents for styling anionic, cationic, amphoteric or nonionic polymers with hair-fixing properties, and mixtures thereof.

16. Composition according to any one of Claims 1 to 15, **characterized in that** the grafted silicone polymer is dissolved in the aqueous medium of the composition or used in the form of an aqueous dispersion of particles.

17. Composition according to any one of Claims 1 to 16, **characterized in that** the keratin substance is human hair.

18. Composition according to any one of Claims 1 to 17, **characterized in that** it is a hair product for maintaining and/or shaping the hair and/or for conditioning the hair.

19. Non-therapeutic process for maintaining and/or shaping the hair and/or for conditioning the hair, **characterized in that** it consists in applying a composition as defined according to any one of Claims 1 to 18 to the hair.

## Patentansprüche

1. Wäßrige Zusammensetzung für die Behandlung von Keratinmaterialien, die in einem Aerosolbehälter verpackt ist, die an der Austrittsöffnung des Behälters einen Lack erzeugt, **dadurch gekennzeichnet, dass** sie
(a) mindestens ein gepfropftes Siliconpolymer mit Polysiloxanrückgrat, das mit nicht-siliconhaltigen organischen Monomeren gepfropft ist;
(b) mindestens 30 Gew.-% Wasser;
(c) mindestens ein Treibmittel;
(d) eine Menge an organischem Lösemittel, die im Bereich von 0 bis 25 Gew.-% liegt, wobei die in Gewichtsprozent gemachten Angaben bezogen auf das Gesamtgewicht der Zusammensetzung in dem Aerosol definiert sind,
enthält und dass das gepfropfte Siliconpolymer (a) unter den Siliconpolymeren ausgewählt ist, die in ihrer Struktur die Einheit der folgenden Formel (IV) 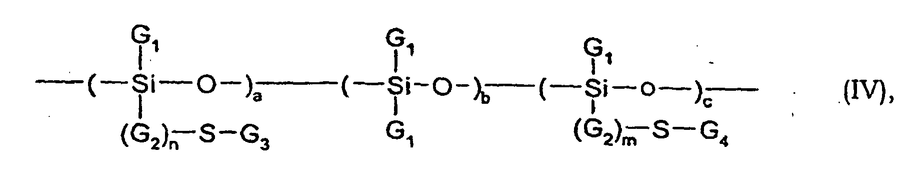 enthalten, in der bedeuten: die Reste G₁, gleich oder verschieden, Wasserstoff oder einen C₁₋₁₀-Alkylrest oder einen Phenylrest; die Reste G₂, gleich oder verschieden, eine C₂₋₁₀-Alkylengruppe; G₃ einen Polymerrest, der bei der (Homo)polymerisation mindestens eines ethylenisch ungesättigten anionischen Monomers entsteht; G₄ einen Polymerrest, der bei der (Homo)polymerisation mindestens eines ethylenisch ungesättigten hydrophoben Monomers entsteht; m und n die Zahl 0 oder 1; a eine ganze Zahl, die im Bereich von 0 bis 50 liegt; b eine ganze Zahl, die im Bereich von 10 bis 350 liegen kann, c eine ganze Zahl, die im Bereich von 0 bis 50 liegt; mit der Maßgabe, dass einer der Parameter a und c verschieden von 0 ist.

2. Zusammensetzung nach Anspruch 1, **dadurch gekennzeichnet, dass** die Einheit der Formel (IV) mindestens eines der folgenden Merkmale aufweist:
- die Reste G₁ bedeuten einen C₁₋₁₀-Rest;
- n ist von Null verschieden, und die Reste G₂ bedeuten einen zweiwertigen C₁₋₃-Rest;
- G₃ stellt einen Polymerrest dar, der bei der (Homo)polymerisation mindestens eines Monomers vom Typ der ethylenisch ungesättigten Carbonsäuren entsteht;
- G₄ stellt einen Polymerrest dar, der bei der (Homo)polymerisation mindestens eines Monomers vom (C₁₋₁₀)-Alkyl(meth)acrylat-Typ entsteht.

3. Zusammensetzung nach Anspruch 1 oder 2, **dadurch gekennzeichnet, dass** die Einheit der Formel (IV) gleichzeitig die folgenden Merkmale aufweist:
- die Reste G₁ bedeuten Methylreste;
- n ist von Null verschieden, und die Reste G₂ sind Propylenreste;
- G₃ stellt einen Polymerrest dar, der bei der (Homo)polymerisation mindestens einer Acrylsäure und/oder Methacrylsäure entsteht;
- G₄ stellt einen Polymerrest dar, der bei der (Homo)polymerisation mindestens eines Monomers vom Isobutyl(meth)acrylat-Typ oder Methyl(meth)acrylat-Typ entsteht.

4. Zusammensetzung nach einem der Ansprüche 1 bis 3, **dadurch gekennzeichnet, dass** das Zahlenmittel des Molekulargewichts des Polymers mit Polysiloxanrückgrat, das mit nicht-siliconhaltigen organischen Monomeren gepfropft ist, im Bereich von etwa 10000 bis 1000000 und noch bevorzugter im Bereich von etwa 10000 bis 100000 liegt.

5. Zusammensetzung nach einem der Ansprüche 1 bis 4, **dadurch gekennzeichnet, dass** das oder die gepfropften Siliconpolymere in einer Menge verwendet werden, die im Bereich von 0,01 bis 20 Gew.-%, bezogen auf das Gesamtgewicht der Zusammensetzung, und vorzugsweise im Bereich von 0,1 bis 15 Gew.-% und noch bevorzugter 0,5 bis 10 Gew.-% liegt.

6. Zusammensetzung nach einem der Ansprüche 1 bis 5, **dadurch gekennzeichnet, dass** sie 45 bis 100 % Wasser, bezogen auf das Gesamtgewicht der Zusammensetzung in dem Aerosol, und noch bevorzugter 60 bis 99,5 % Wasser enthält.

7. Zusammensetzung nach einem der Ansprüche 1 bis 6, **dadurch gekennzeichnet, dass** das organische Lösemittel unter den C₁₋₄-Alkoholen ausgewählt ist.

8. Zusammensetzung nach einem der Ansprüche 1 bis 7, **dadurch gekennzeichnet, dass** sie 0 bis 10 % organisches Lösemittel, bezogen auf das Gesamtgewicht der Zusammensetzung in dem Aerosol, und noch bevorzugter 0 bis 5 % organisches Lösemittel enthält.

9. Zusammensetzung nach einem der Ansprüche 1 bis 8, **dadurch gekennzeichnet, dass** sie kein organisches Lösemittel enthält.

10. Zusammensetzung nach einem der Ansprüche 1 bis 9, **dadurch gekennzeichnet, dass** das Treibmittel unter den flüchtigen Kohlenwasserstoffen, den chlorierten und/oder fluorierten Kohlenwasserstoffen und ihren Gemischen, Kohlendioxidgas, Distickstoffoxid, Dimethylether, Stickstoff, Druckluft und deren Gemischen ausgewählt ist.

11. Zusammensetzung nach einem der Ansprüche 1 bis 10, **dadurch gekennzeichnet, dass** es sich bei dem Treibmittel um Dimethylether handelt.

12. Zusammensetzung nach einem der Ansprüche 1 bis 11, **dadurch gekennzeichnet, dass** das Treibmittel in Konzentrationen enthalten ist, die vorzugsweise im Bereich von 10 bis 68 Gew.-%, bezogen auf das Gesamtgewicht der Zusammensetzung in dem Aerosolbehälter, und noch bevorzugter im Bereich von 25 bis 50 Gew.-% liegen.

13. Zusammensetzung nach einem der Ansprüche 1 bis 12, **dadurch gekennzeichnet, dass** die Konzentration an flüchtigen organischen Verbindungen (VOC) kleiner als oder gleich 55 Gew.-%, bezogen auf das Gesamtgewicht der als Aerosol verpackten Zusammensetzung, ist.

14. Zusammensetzung nach einem der Ansprüche 1 bis 13, **dadurch gekennzeichnet, dass** der pH-Wert im Bereich von 2 bis 9 und insbesondere 3 bis 8 liegt.

15. Zusammensetzung nach einem der Ansprüche 1 bis 14, **dadurch gekennzeichnet, dass** sie zusätzlich einen herkömmlichen kosmetischen Zusatzstoff enthält, der unter den Konservierungsmitteln, den reizlindernden Mitteln, den Maskierungsmitteln, den Parfüms, den Mitteln zur Modifizierung der Viskosität, den Perlglanzmitteln, den Hydratisierungsmitteln, den Antischuppenmitteln, den Antiseborrhöika, den Sonnenschutzfiltern, den Konditionierern für die Haare, den Antioxidantien, den Proteinen, den Vitaminen, den Siliconen, den Alkalisierungsmitteln und den Säuerungsmitteln für die frisierenden anionischen, kationischen, amphoteren oder nichtionischen Polymeren mit haarfestigenden Eigenschaften, und deren Gemischen ausgewählt ist.

16. Zusammensetzung nach einem der Ansprüche 1 bis 15, **dadurch gekennzeichnet, dass** das gepfropfte Siliconpolymer in dem wäßrigen Medium der Zusammensetzung gelöst ist oder in Form einer wäßrigen Partikeldispersion verwendet wird.

17. Zusammensetzung nach einem der Ansprüche 1 bis 16, **dadurch gekennzeichnet, dass** es sich bei den Keratinmaterialien um menschliche Haare handelt.

18. Zusammensetzung nach einem der Ansprüche 1 bis 17, **dadurch gekennzeichnet, dass** sie ein Mittel zur Haarbehandlung für die Frisurenerhaltung und/oder die Frisurengestaltung und/oder die Konditionierung der Haare ist.

19. Nicht-therapeutisches Verfahren für den Halt und/oder die Gestaltung des Haars und/ oder die Konditionierung der Haare, **dadurch gekennzeichnet, dass** es darin besteht, auf die Haare eine wie in einem der Ansprüche 1 bis 18 definierte Zusammensetzung aufzutragen.
